# FASCICULE DE BREVET EUROPEEN

(11) **EP 0 667 912 B1**
(45) Date de publication et mention de la délivrance du brevet: **02.07.2008**
(21) Numéro de dépôt: 94922889.4
(22) Date de dépôt: 08.07.1994
(51) Int. Cl.: C12N 15/861, C12N 5/10, A61K 48/00

(54) **VECTEURS ADENOVIRAUX DEFECTIFS ET UTILISATION EN THERAPIE GENIQUE**
DEFEKTE ADENOVIRUS-VEKTOREN UND DEREN VERWENDUNG IN DER GENTHERAPIE
DEFECTIVE ADENOVIRUS VECTORS AND USE THEREOF IN GENE THERAPY

(30) Priorité: 13.07.1993 FR 9308596; 18.04.1994 FR 9404590
(43) Date de publication de la demande: 23.08.1995
(73) Titulaire: CENTELION, 94400 Vitry Sur Seine (FR)
(72) Inventeur: PERRICAUDET, Michel, F-28320 Ecrosnes (FR); VIGNE, Emmanuelle, F-94200 Ivry-sur-Seine (FR); YEH, Patrice, F-75005 Paris (FR)
(74) Mandataire: Bouvet, Philippe
(86) Numéro de dépôt international: PCT/FR1994/000851
(87) Numéro de publication internationale: WO 1995/002697

(56) Documents cités:
- WO-A-93/06223
- WO-A-94/12649
- WO-A-94/28152
- WO-A-94/28938
- JOURNAL OF VIROLOGY vol. 63, no. 6 , Juin 1989 pages 2709 - 2717 BABISS, L.E. 'The cellular transcription factor E2f requires viral E1A and E4 gene products for increased DNA-binding activity and functions to stimulate adenovirus E2A gene expression'
- HUMAN GENE TRANSFER vol. 219 , 1991 pages 51 - 61 STATFORD-PERRICAUDET, L. & PERRICAUDET, M. 'Gene transfer into animals: the promise of adenovirus'
- PROCEEDINGS OF THE NATIONAL ACADEMY OF SCIENCES OF USA. vol. 80 , Septembre 1983 , WASHINGTON US pages 5383 - 5386 WEINBERG, D.H. & KETNER, G. 'A cell line that supports the growth of a defective early region 4 deletion mutant of human adenovirus type 2'
- NUCLEIC ACIDS RESEARCH. vol. 17, no. 8 , 1989 , ARLINGTON, VIRGINIA US pages 3037 - 3047 KETNER, G. ET AL. 'Complementation of adenovirus E4 mutants by transient expression of E4 cDNA and deletion plasmids'

## Description

La présente invention concerne de nouveaux vecteurs viraux, leur préparation et leur utilisation en thérapie génique. Elle concerne également les compositions pharmaceutiques contenant lesdits vecteurs viraux. Plus particulièrement, la présente invention concerne des adénovirus recombinants comme vecteurs pour la thérapie génique.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Parmi ces virus, les adénovirus présentent certaines propriétés intéressantes pour une utilisation en thérapie génique. Notamment, ils ont un spectre d'hôte assez large, sont capables d'infecter des cellules quiescentes, ne s'intègrent pas au génome de la cellule infectée, et n'ont pas été associés à ce jour à des pathologies importantes chez l'homme.

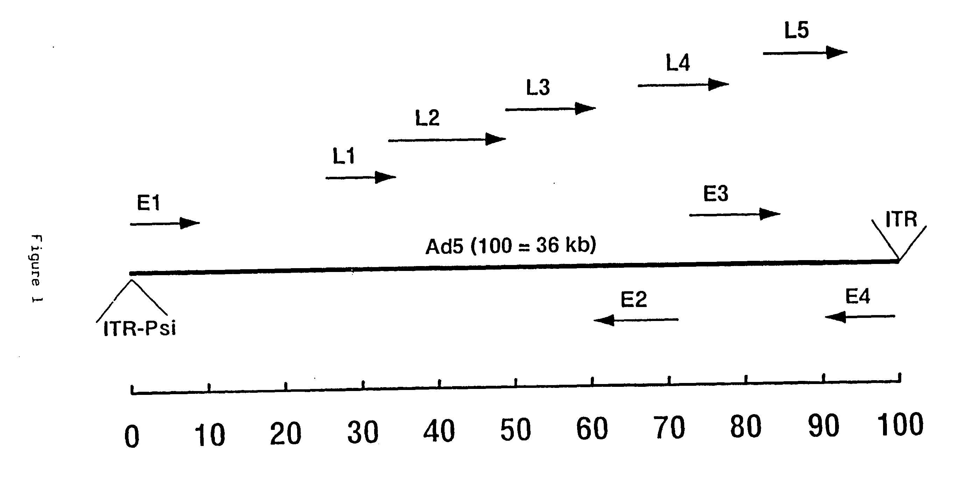

Les adénovirus sont des virus à ADN double brin linéaire d'une taille de 36 kb environ. Leur génome comprend notamment une séquence inversée répétée (ITR) à leur extrémité, une séquence d'encapsidation, des gènes précoces et des gènes tardifs (Cf figure 1). Les principaux gènes précoces sont les gènes E1 (E1a et E1b), E2, E3 et E4. Les principaux gènes tardifs sont les gènes L1 à L5.

Compte tenu des propriétés des adénovirus mentionnées ci-dessus, ceux-ci ont déjà été utilisés pour le transfert de gènes in vivo. A cet effet, différents vecteurs dérivés des adénovirus ont été préparés, incorporant différents gènes (β-gal, OTC, α-1 AT, cytokines, etc). Dans chacune de ces constructions, l'adénovirus a été modifié de manière à le rendre incapable de réplication dans la cellule infectée. Ainsi, les constructions décrites dans l'art antérieur sont des adénovirus délétés des régions E1 (E1a et/ou E1b) et éventuellement E3 au niveau desquelles sont insérées les séquences d'ADN hétérologue (Levrero et al., Gene 101 (1991) 195; Gosh-Choudhury et al., Gene 50 (1986) 161). WO 93/06223 divulgue des adénovirus dérivés de dl324 qui contiennent le gène de la beta-galactosidase et ont des délétions des régions E1 et E3. Néanmoins, les vecteurs décrits dans l'art antérieur présentent de nombreux inconvénients qui limitent leur exploitation en thérapie génique. En particulier, tous ces vecteurs comportent de nombreux gènes viraux dont l'expression in vivo n'est pas souhaitable dans le cadre d'une thérapie génique. De plus, ces vecteurs ne permettent pas l'incorporation de fragments d'ADN de très grande taille, qui peuvent être nécessaires pour certaines applications.

La présente invention permet de remédier à ces inconvénients. La présente invention décrit en effet des adénovirus recombinants pour la thérapie génique, capables de transférer de manière efficace de l'ADN (jusqu'à 30 kb) in vivo, d'exprimer à des niveaux élevés et de manière stable cet ADN in vivo, en limitant tout risque de production de protéines virales, de transmission du virus, de pathogénicité, etc. En particulier, il a été trouvé qu'il est possible de réduire considérablement la taille du génome de l'adénovirus, sans empécher la formation d'une particule virale encapsidée. Ceci est surprenant dans la mesure où il avait été observé dans le cas d'autres virus, par exemple des rétrovirus, que certaines séquences distribuées le long du génome étaient nécessaires pour une encapsidation efficace des particules virales. De ce fait, la réalisation de vecteurs possédant d'importantes délétions internes était fortement limitée. La présente invention montre également que la suppression de l'essentiel des gènes viraux n'empêche pas non plus la formation d'une telle particule virale. De plus, les adénovirus recombinants ainsi obtenus conservent, en dépit des modifications importantes de leur structure génomique, leurs propriétés avantageuses de fort pouvoir infectieux, de stabilité in vivo, etc.

Les vecteurs de l'invention sont particulièrement avantageux puisqu'ils permettent l'incorporation de séquences d'ADN désirées de très grande taille. Il est ainsi possible d'insérer un gène d'une longueur supérieure à 30 kb. Ceci est particulièrement intéressant pour certaines pathologies dont le traitement nécessite la co-expression de plusieurs gènes, ou l'expression de très grands gènes. Ainsi par exemple, dans le cas de la dystrophie musculaire, il n'était pas possible jusqu'à présent de transférer l'ADNc correspondant au gène natif responsable de cette pathologie (gène de la dystrophine) en raison de sa grande taille (14 kb).

Les vecteurs de l'invention sont également très avantageux puisqu'ils possèdent très peu de régions virales fonctionnelles et que, de ce fait, les risques inhérents à l'utilisation de virus comme vecteurs en thérapie génique tels que l'immunogénicité, la pathogénicité, la transmission, la réplication, la recombinaison, etc sont fortement réduits voire supprimés.

La présente invention fournit ainsi des vecteurs viraux particulièrement adaptés au transfert et à l'expression in vivo de séquences d'ADN désirées.

L'objet de la présente invention concerne donc un adénovirus recombinant défectif comprenant :
- les séquences ITR,
- une séquence permettant l'encapsidation,
- une séquence d'ADN hétérologue,
- une région portant le gène E2,
et dans lequel :
- le gène E1 est non fonctionnel et
- au moins le gène E4, est non fonctionnel.

Au sens de la présente invention, le terme "adénovirus défectif" désigne un adénovirus incapable de se répliquer de façon autonome dans la cellule cible. Généralement, le génome des adénovirus défectifs selon la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence d'ADN hétérologue.

Les séquences inversées répétées (ITR) constituent l'origine de réplication des adénovirus. Elles sont localisées aux extrémités 3' et 5' du génome viral (Cf figure 1), d'où elles peuvent être isolées aisément selon les techniques classiques de biologie moléculaire connues de l'homme du métier. La séquence nucléotidique des séquences ITR des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence ITR gauche correspond à la région comprenant les nucléotides 1 à 103 du génome.

La séquence d'encapsidation (également désignée séquence Psi) est nécessaire à l'encapsidation de l'ADN viral. Cette région doit donc être présente pour permettre la préparation d'adénovirus recombinants défectifs selon l'invention. La séquence d'encapsidation est localisée dans le génome des adénovirus, entre 1TTR gauche (5') et le gène E1 (Cf figure 1). Elle peut être isolée ou synthétisée artificiellement par les techniques classiques de biologie moléculaire. La séquence nucléotidiques de la séquence d'encapsidation des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence d'encapsidation correspond à la région comprenant les nucléotides 194 à 358 du génome.

Il existe différents sérotypes d'adénovirus, dont la structure et les propriétés varient quelque peu. Néanmoins, ces virus présentent une organisation génétique comparable, et les enseignements décrits dans la présente demande peuvent être aisément reproduits par l'homme du métier pour tout type d'adénovirus.

Les adénovirus de l'invention peuvent être d'origine humaine, animale, ou mixte (humaine et animale).

Concernant les adénovirus d'origine humaine, on préfère utiliser ceux classés dans le groupe C. Plus préférentiellement, parmi les différents sérotypes d'adénovirus humain, on préfère utiliser dans le cadre de la présente invention les adénovirus de type 2 ou 5 (Ad 2 ou Ad 5).

Comme indiqué plus haut, les adénovirus de l'invention peuvent également être d'origine animale, ou comporter des séquences issues d'adénovirus d'origine animale. La demanderesse a en effet montré que les adénovirus d'origine animale sont capables d'infecter avec une grande efficacité les cellules humaines, et qu'ils sont incapables de se propager dans les cellules humaines dans lesquelles ils ont été testés (Cf demande FR 93 05954). La demanderesse a également montré que les adénovirus d'origine animale ne sont nullement trans-complémentés par des adénovirus d'origine humaine, ce qui élimine tout risque de recombinaison et de propagation in vivo, en présence d'un adénovirus humain, pouvant conduire à la formation d'une particule infectieuse. L'utilisation d'adénovirus ou de régions d'adénovirus d'origine animale est donc particulièrement avantageuse puisque les risques inhérents à l'utilisation de virus comme vecteurs en thérapie génique sont encore plus faibles.

Les adénovirus d'origine animale utilisables dans le cadre de la présente invention peuvent être d'origine canine, bovine, murine, (exemple : Mav1, Beard et al., Virology 75 (1990) 81), ovine, porcine, aviaire ou encore simienne (exemple : SAV). Plus particulièrement, parmi les adénovirus aviaires, on peut citer les sérotypes 1 à 10 accessibles à l'ATCC, comme par exemple les souches Phelps (ATCC VR-432), Fontes (ATCC VR-280), P7-A (ATCC VR-827), IBH-2A (ATCC VR-828), J2-A (ATCC VR-829), T8-A (ATCC VR-830), K-11 (ATCC VR-921) ou encore les souches référencées ATCC VR-831 à 835. Parmi les adénovirus bovins, on peut utiliser les différents sérotypes connus, et notamment ceux disponibles à l'ATCC (types 1 à 8) sous les références ATCC VR-313, 314, 639-642, 768 et 769. On peut également citer les adénovirus murins FL (ATCC VR-550) et E20308 (ATCC VR-528), l'adénovirus ovin type 5 (ATCC VR-1343), ou type 6 (ATCC VR-1340); l'adénovirus porcin 5359), ou les adénovirus simiens tels que notamment les adénovirus référencée à l'ATCC sous les numéros VR-591-594, 941-943, 195-203, etc.

De préférence, parmi les différents adénovirus d'origine animale, on utilise dans le cadre de l'invention des adénovirus ou des régions d'adénovirus d'origine canine, et notamment toutes les souches des adénovirus CAV2 [souche manhattan ou A26/61 (ATCC VR-800) par exemple]. Les adénovirus canins ont fait l'objet de nombreuses études structurales. Ainsi, des cartes de restriction complètes des adénovirus CAV1 et CAV2 ont été décrites dans l'art antérieur (Spibey et al., J. Gen. Virot. 70 (1989) 165), et les gènes E1a, E3 ainsi que les séquences ITR ont été clonés et séquencés (voir notamment Spibey et al., Virus Res. 14 (1989) 241; Linné, Virus Res. 23 (1992) 119, WO 91/11525).

Comme indiqué ci-avant, les adénovirus de la présente invention comportent une séquence d'ADN hétérologue. La séquence d'ADN hétérologue désigne toute séquence d'ADN introduite dans le virus recombinant, dont le transfert et/ou l'expression dans la cellule cible est recherchée.

En particulier, la séquence d'ADN hétérologue peut comporter un ou plusieurs gènes thérapeutiques et/ou un ou plusieurs gènes codant pour des peptides antigéniques.

Les gènes thérapeutiques qui peuvent ainsi être transférés sont tout gène dont la transcription et éventuellement la traduction dans la cellule cible génèrent des produits ayant un effet thérapeutique.

Il peut s'agir en particulier de gènes codant pour des produits protéiques ayant un effet thérapeutique. Le produit protéique ainsi codé peut être une protéine, un peptide, un acide aminé, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule lui permettant de lutter contre une pathologie.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc.

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140308.

Comme indiqué plus haut, la séquence d'ADN hétérologue peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, la séquence d'ADN hétérologue comprend également des séquences permettant l'expression du gène thérapeutique et/ou du gêne codant pour le peptide antigénique dans la cellule infectée. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP, CMV, RSV, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Par ailleurs, la séquence d'ADN hétérologue peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Comme indiqué ci-avant, les vecteurs de l'invention possèdent une région portant le gène E2, et au moins le gène E1 et le gène E4 sont non fonctionnels. Le gène viral considéré peut être rendu non fonctionnel par toute technique connue de l'homme du métier, et notamment par suppression, substitution, délétion, ou addition d'une ou plusieurs bases dans le ou les gènes considères. De telles modifications peuvent être obtenues in vitro (sur de l'ADN isolé) ou in situ, par exemple, au moyen des techniques du génie génétique, ou encore par traitement au moyen d'agents mutagènes.

Parmi les agents mutagènes, on peut citer par exemple les agents physiques tels que les rayonnements énergétiques (rayons X, g, ultra violet, etc..), ou les agents chimiques capables de réagir avec différents groupements fonctionnels des bases de l'ADN, et par exemple les agents alkylants [éthylméthane sulfonate (EMS), N-méthyl-N-nitro-N-nitrosoguanidine, N-nitroquinoléine-1-oxyde (NQO)], les agents bialkylants, les agents intercalants, etc.

Par délétion on entend au sens de l'invention toute suppression du gène considéré. Il peut s'agir notamment de tout ou partie de la région codante dudit gène, et/ou de tout ou partie de la région promotrice de la transcription dudit gène. La suppression peut être effectuée par digestion au moyen d'enzymes de restriction appropriées, puis ligature, selon les techniques classiques de biologie moléculaire, ainsi qu'illustré dans les exemples.

Les modifications génétiques peuvent également être obtenues par disruption génique, par exemple selon le protocole initialement décrit par Rothstein [Meth. Enzymol. 101 (1983) 202]. Dans ce cas, tout ou partie de la séquence codante est préférentiellement perturbée pour permettre le remplacement, par recombinaison homologue, de la séquence génomique par une séquence non fonctionnelle ou mutante.

La ou lesdites modifications génétiques peuvent être localisées dans la partie codante du gène concerné, ou en dehors de la région codante, et par exemple dans les régions responsables de l'expression et/ou de la régulation transcriptionelle desdits gènes. Le caractère non fonctionnel desdits gènes peut donc se manifester par la production d'une protéine inactive en raison de modifications structurales ou conformationelles, par l'absence de production, par la production d'une protéinee ayant une activité altérée, ou encore par la production de la protéine naturelle à un niveau atténué ou selon un mode de régulation désiré.

Par ailleurs, certaines altérations telles que des mutations ponctuelles sont par nature capables d'être corrigées ou atténuées par des mécanismes cellulaires. De telles altérations génétiques ont alors un intérêt limité au niveau industriel. Il est donc particulièrement préféré que le caractère non fonctionnel soit parfaitement stable ségrégationellement et/ou non-réversible..

De préférence, le gène est non fonctionnel en raison d'une délétion partielle ou totale.

Préférentiellement, les adénovirus recombinants défectifs de l'invention sont dépourvus de gènes tardifs d'adénovirus.

Sont aussi divulgués ici un adénovirus recombinant défectif comprenant :
- les séquences ITR,
- une séquence permettant l'encapsidation,
- une séquence d'ADN hétérologue, et
- une région portant le gène ou une partie du gène E2 ;
ainsi/qu'un adénovirus recombinant défectif comprenant :
- les séquences ITR,
- une séquence permettant l'encapsidation,
- une séquence d'ADN hétérologue, et
- une région portant le gène ou une partie du gène E4.

Dans un mode particulierement avantageux, les vecteurs de l'invention possèdent en outre un gène E3 fonctionnel sous controle d'un promoteur hétérologue. Plus préférentiellement, les vecteurs possèdent une partie du gène E3 permettant l'expression de la protéine gp19K.

Les adénovirus recombinants défectifs selon l'invention peuvent être préparés de différentes façons.

Une première méthode consiste à transfecter l'ADN du virus recombinant défectif préparé in vitro (soit par ligature, soit sous forme de plasmide) dans une lignée cellulaire compétente, c'est-à-dire portant en trans toutes les fonctions nécessaires à la complémentation du virus défectif. Ces fonctions sont préférentiellement intégrées dans le génome de la cellule, ce qui permet d'éviter les risques de recombinaison, et confère une stabilité accrue à la lignée cellulaire. La préparation de telles lignées cellulaires est décrite dans les exemples.

Une seconde approche consiste à co-transfecter dans une lignée cellulaire apppropriée l'ADN du virus recombinant défectif préparé in vitro (soit par ligature, soit sous forme de plasmide) et l'ADN d'un virus helper. Selon cette méthode, il n'est pas nécessaire de disposer d'une lignée cellulaire compétente capable de complémenter toutes les fonctions défectives de l'adénovirus recombinant. Une partie de ces fonctions est en effet complémentée par le virus helper. Ce virus helper doit lui-même être défectif et la lignée cellulaire porte en trans les fonctions nécessaires à sa complémentation. La préparation d'adénovirus recombinants défectifs de l'invention selon cette méthode est également illustrée dans les exemples.

Parmi les lignées cellulaires utilisables dans le cadre de cette seconde approche, on peut citer notamment la lignée de rein embryonnaire humain 293, les cellules KB, les cellules Hela, MDCK, GHK, etc (Cf exemples).

Ensuite, les vecteurs qui se sont multipliés sont récupérés, purifiés et amplifiés selon les techniques classiques de biologie moléculaire.

La présente invention concerne donc aussi les lignées cellulaires infectables par les adénovirus, comprenant, intégrées dans leur génome, les fonctions nécessaires à la complémentation d'un adénovirus recombinant défectif tel que décrit précédemment. En particulier, elle concerne les lignées cellulaires comportant, intégrées dans leur génome, les régions E1 et E2 (notamment la région codant pour la protéine 72K), et/ou E4 et/ou le gène du récepteur aux glucocorticoïdes. Préférentiellement, ces lignées sont obtenues à partie de la lignée 293 ou gm DBP6.

La présente invention concerne également toute composition pharmaceutique comprenant un ou plusieurs adénovirus recombinants défectifs tels que décrits précédemment. Les compositions pharmaceutiques de l'invention peuvent être formulées en vue d'une administration par voie topique, orale, parentérale, intranasale, intraveineuse, intramusculaire, sous-cutanée, intraoculaire, transdermique, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 5 jours, du nombre de plages de cellules infectées. Les techniques de détermination du titre pfu d'une solution virale sont bien documentées dans la littérature.

Selon la séquence d'ADN hétérologue insérée, les adénovirus de l'invention peuvent être utilisés pour le traitement ou la prévention de nombreuses pathologies, incluant les maladies génétiques (dystrophie, fibrose cystique, etc), les maladies neurogégénératives (alzheimer, parkinson, ALS, etc), les cancers, les pathologies liées aux désordres de la coagulation ou aux dyslipoprotéinémies, les pathologies liées aux infections virales (hépatites, SIDA, etc), etc.

La présente invention sera plus complètement décrite à l'aide des exemples qui suivent, qui doivent être considérés comme illustratifs et non limitatifs.

### Légende des figures

Figure 1 : Organisation génétique de l'adénovirus Ad5. La séquence complète de l'Ad5 est disponible sur base de données et permet à l'homme du métier de sélectionner ou de créer tout site de restriction, et ainsi d'isoler toute région du génome.

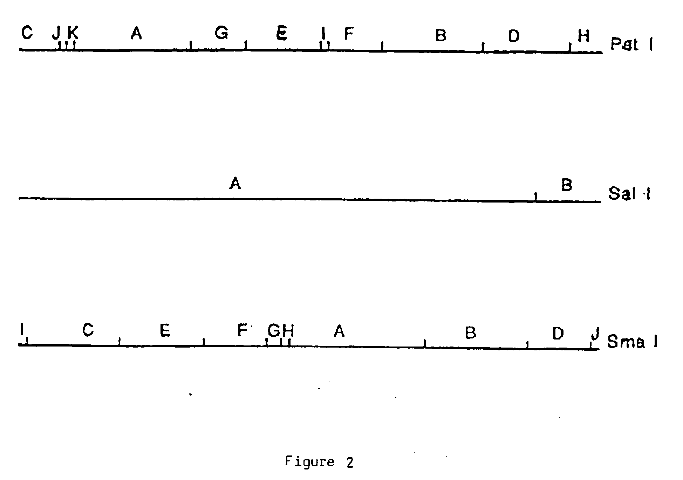

Figure 2 : Carte de restriction de l'adénovirus CAV2 souche Manhattan (d'après Spibey et al précité).
Figure 3 : Construction de virus défectifs de l'invention par ligature.
Figure 4 : Construction d'un virus recombinant portant le gène E4.
Figure 5 : Construction d'un virus recombinant portant le gène E2.
Figure 6 : Construction et représentation du plasmide pPY32.
Figure 7 : Représentation du plasmide pPY55.
Figure 8 : Représentation du plasmide p2.
Figure 9 : Représentation du plasmide intermédiaire utilisé pour la construction du plasmide pITRL5-E4.
Figure 10 : Représentation du plasmide pITRL5-E4.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Lignées cellulaires utilisées

Dans les exemples qui suivent, les lignées cellulaires suivantes ont ou peuvent être utilisées :
- Lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %).
- Lignée de cellules humaines KB : Issue d'un carcinome épidermique humain, cette lignée est accessible à l'ATCC (ref. CCL17) ainsi que les conditions permettant sa culture.
- Lignée de cellules humaines Hela : Issue d'un carcinome de l'épithélium humain, cette lignée est accessible à l'ATCC (ref. CCL2) ainsi que les conditions permettant sa culture.
- Lignée de cellules canines MDCK : Les conditions de culture des cellules MDCK ont été décrites notamment par Macatney et al., Science 44 (1988) 9.
- Lignée de cellules gm DBP6 (Brough et al., Virology 190 (1992) 624).
Cette lignée est constituée de cellules Hela portant le gène E2 d'adénovirus sous le controle du LTR de MMTV.

### EXEMPLES

### Exemple de référence 1:

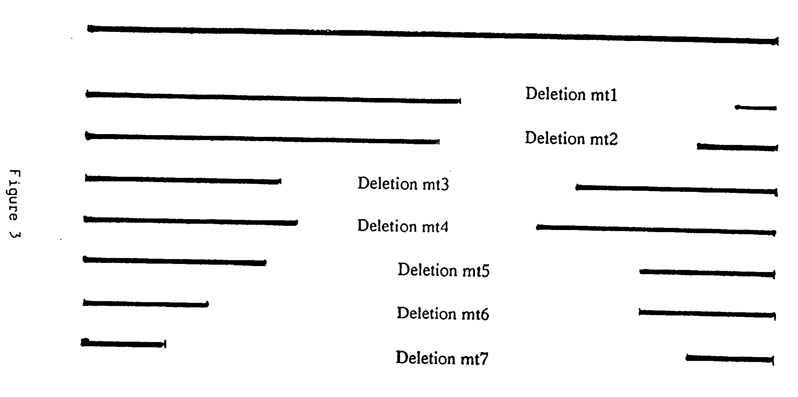

Cet exemple démontre la faisabilité d'un adénovirus recombinant dépourvu de l'essentiel des gènes viraux. Pour cela, une série de mutants de délétion dans l'adénovirus a été construite par ligation in vitro, et chacun de ces mutants a été co-transfecté avec un virus helper dans les cellules KB. Ces cellules ne permettant pas la propagation des virus défectifs pour E1, la transcomplémentation porte sur la région E1.
Les différents mutants de délétion ont été préparés à partir de l'adénovirus Ad5 par digestion puis ligation in vitro. Pour cela, l'ADN viral d'Ad5 est isolé selon la technique décrite par Lipp et al. (J. Virol. 63 (1989) 5133), soumis à digestion en présence de différentes enzymes de restriction (Cf figure 3), puis le produit de la digestion est ligaturé en présence de T4 DNA ligase. La taille des différents mutants de délétion est ensuite controlée sur gel SDS agarose 0,8%. Ces mutants sont ensuite cartographiés (Cf figure 3). Ces différents mutants comportent les régions suivantes :
mt1 : Ligation entre les fragments d'Ad5 0-20642(SauI) et (SauI)33797-35935
mt2 : Ligation entre les fragments d'Ad5 0-19549(NdeI) et (NdeI)31089-35935
mt3 : Ligation entre les fragments d'Ad5 0-10754(AatII) et (AatII)25915-35935
mt4 : Ligation entre les fragments d'Ad5 0-11311(MluI) et (MluI)24392-35935
mt5 : Ligation entre les fragments d'Ad5 0-9462(SalI) et (XhoI)29791-35935
mt6 : Ligation entre les fragments d'Ad5 0-5788(XhoI) et (XhoI)29791-35935
mt7 : Ligation entre les fragments d'Ad5 0-3665(SphI) et (SphI)31224-35935

Chacun des mutants préparés ci-dessus a été co-transfecté avec l'ADN viral de Ad.RSVβGal (Stratford-Perricaudet et al., J.Clin.Invest. 90 (1992) 626) dans les cellules KB, en présence de phosphate de calcium. Les cellules ont été récoltées 8 jours après la transfection, et les surnageants de culture ont été récoltés puis amplifiés sur cellules KB jusqu'à l'obtention de stocks de 50 boites pour chaque transfection. A partir de chaque échantillon, l'ADN épisomique a été isolé et séparé sur gradient de chlorure de césium. Deux bandes distinctes de virus ont été observées dans chaque cas, prélevées et analysées. La plus lourde correspond à l'ADN viral de Ad.RSVβGal, et la plus légère à l'ADN du virus recombinant généré par ligation (figure 3). Le titre obtenu pour cette dernière est d'environ 10⁸ pfu/ml.

Une seconde série de mutants de délétion dans l'adénovirus a été construite par ligation in vitro selon la même méthodologie. Ces différents mutants comportent les régions suivantes :
mt8 : Ligation entre les fragments 0-4623(ApaI) d'Ad RSVβGal et (ApaI)31909-35935 d'Ad5.
mt9 : Ligation entre les fragments 0-10178(BglII) d'Ad RSVβGal et (BamHI)21562-35935 d"Ad5.

Ces mutants, portant le gène LacZ sous controle du promoteur LTR du virus RSV, sont ensiute co-transfectés dans les cellules 293 en présence de l'ADN viral de H2dl808 (Weinberg et al., J. Virol. 57 (1986) 833), qui est délété de la région E4. Selon cette seconde technique, la transcomplémentation porte sur E4 et non plus sur E1. Cette technique permet ainsi de générer, comme décrit ci-dessus, des virus recombinants ne possédant comme gène viral que la région E4.

### Exemple de référence 2:

Cet exemple décrit la préparation d'adénovirus recombinants défectifs par co-transfection, avec un virus helper, de l'ADN du virus recombinant incorporé à un plasmide.

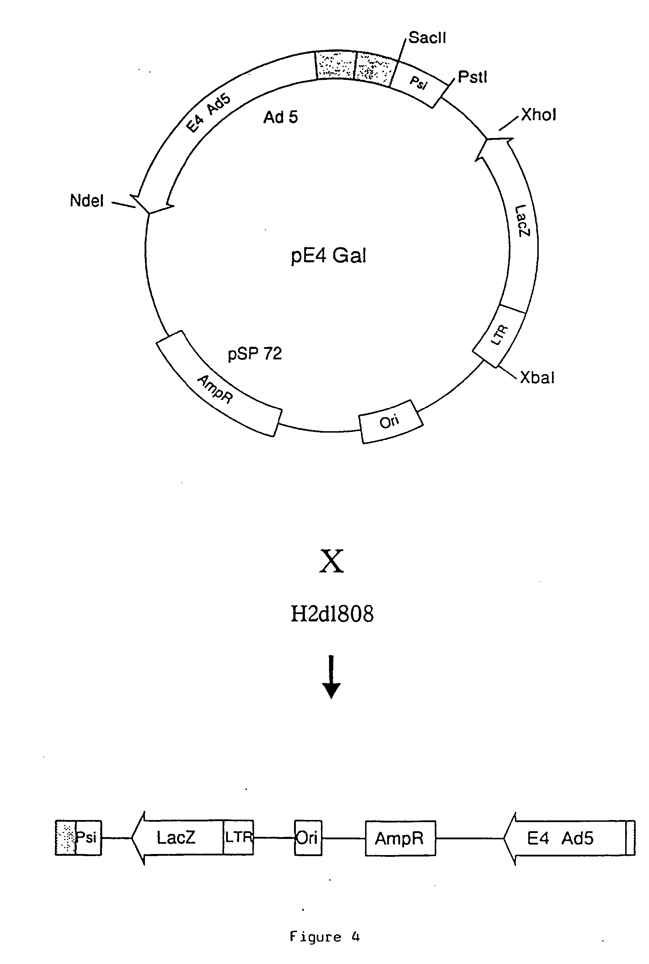

Pour cela, un plasmide portant les ITR jointives de l'Ad5, la séquence d'encapsidation, le gène E4 sous controle de son propre promoteur et, comme gène hétérologue, le gène LacZ sous controle du promoteur LTR du virus RSV a été construit (figure 4). Ce plasmide, désigné pE4Gal a été obtenu par clonage et ligature des fragments suivants (voir figure 4) :
- fragment HindIII-SacII issu du plasmide pFG144 (Graham et al, EMBO J. 8 (1989) 2077). Ce fragment porte les séquences ITR de l'Ad5 en tête à queue et la séquence d'encapsidation : fragment HindIII (34920)-SacII (352).
- fragment de l'Ad5 compris entre les sites SacII (localisé au niveau de la paire de bases 3827) et PstI (localisé au niveau de la paire de bases 4245);
- fragment de pSP 72 (Promega) compris entre les sites PstI (pb 32) et SalI (pb 34);
- fragment XhoI-XbaI du plasmide pAdLTR GalIX décrit dans Stratford-Perricaudet et al (JCI 90 (1992) 626). Ce fragment porte le gène LacZ sous controle du LTR du virus RSV.
- fragment XbaI (pb 40) - NdeI (pb 2379) du plasmide pSP 72;
- fragment NdeI (pb 31089) - HindIII (pb 34930) de l'Ad5. Ce fragment localisé dans l'extrémité droite du génome de l'Ad5 contient la région E4 sous controle de son propre promoteur. Il a été cloné au niveau des sites NdeI (2379) du plasmide pSP 72 et HindIII du premier fragment.

Ce plasmide a été obtenu par clonage des différents fragments dans les régions indiquées du plasmide pSP 72. Il est entendu que des fragments équivalents peuvent être obtenus par l'homme du métier à partir d'autres sources.

Le plasmide pE4Gal est ensuite co-transfecté avec l'ADN du virus H2d1808 dans les cellules 293 en présence de phosphate de calcium. Le virus recombinant est ensuite préparé comme décrit dans l'exemple 1. Ce virus porte, comme seul gène viral, le gène E4 de l'adénovirus Ad5 (figure 4). Son génome a une taille de 12 kb environ, ce qui permet l'insertion d'ADN hétérologue de très grande taille (jusqu'à 20 kb). Ainsi, l'homme du métier peut aisément remplacer le gène LacZ par tout autre gène thérapeutique tels que ceux mentionnés plus haut. Par ailleurs, ce virus comporte certaines séquences issues du plasmide pSP 72, qui peuvent être éliminées par les techniques classiques de biologie moléculaires si nécessaire.

### Exemple 3

Cet exemple décrit la préparation d'un autre adénovirus recombinant défectif selon l'invention par co-transfection, avec un virus helper, de l'ADN du virus recombinant incorporé à un plasmide.

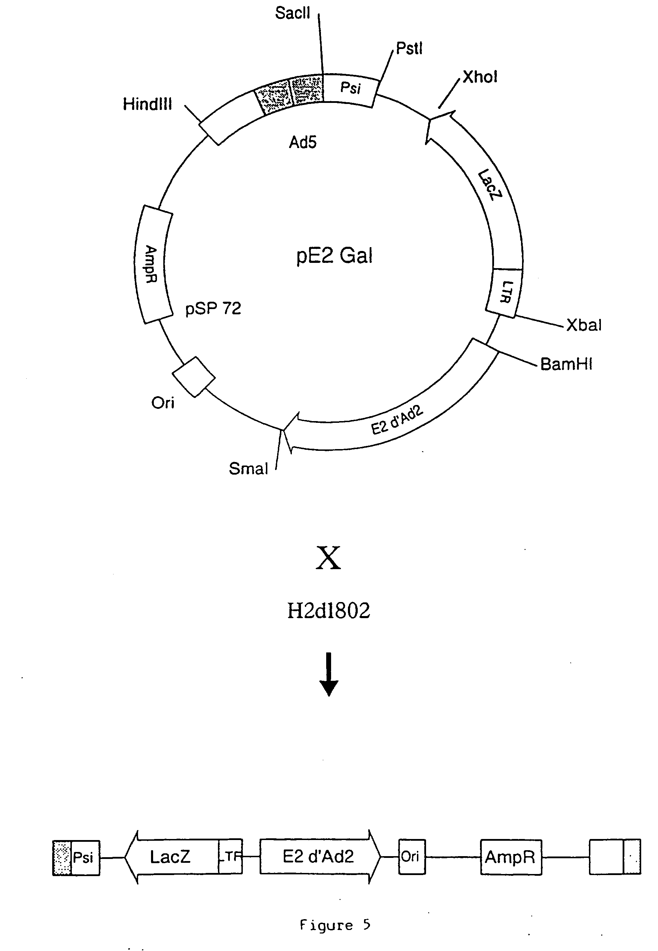

Pour cela, un plasmide portant les ITR jointives de l'Ad5, la séquence d'encapsidation, le gène E2 de l'Ad2 sous controle de son propre promoteur et, comme gène hétérologue, le gène LacZ sous controle du promoteur LTR du virus RSV a été construit (figure 5). Ce plasmide, désigné pE2Gal a été obtenu par clonage et ligature des fragments suivants (voir figure 5) :
- fragment HindIII-SacII issu du plasmide pFG144 (Graham et al, EMBO J. 8 (1989) 2077). Ce fragment porte les séquences ITR de l'Ad5 en tête à queue et la séquence d'encapsidation : fragment HindIII (34920)-SacII (352). Il a été cloné, avec le fragment suivant, au niveau des sites HindIII (16) - PstI (32) du plasmide pSP 72.
- fragment de l'Ad5 compris entre les sites SacII (localisé au niveau de la paire de bases 3827) et PstI (localisé au niveau de la paire de bases 4245). Ce fragment a été cloné au niveau du site SacII du fragment précédent et du site PstI (32) du plasmide pSP 72.
- fragment de pSP 72 (Promega) compris entre les sites PstI (pb 32) et SalI (pb 34);
- fragment XhoI-XbaI du plasmide pAdLTR GalIX décrit dans Stratford-Perricaudet et al (JCI 90 (1992) 626). Ce fragment porte le gène LacZ sous controle du LTR du virus RSV. Il a été cloné au niveau des sites SalI (34) et XbaI du plasmide pSP 72.
- fragment de pSP 72 (Promega) compris entre les sites XbaI (pb 34) et BamHI (pb 46);
- fragment BamHI (pb 21606) - SmaI (pb 27339) de l'Ad2. Ce fragment du génome de l'Ad2 contient la région E2 sous controle de son propre promoteur. Il a été cloné au niveau des sites BamHI (46) et EcoRV du plasmide pSP 72.
- fragment EcoRV (pb 81) - HindIII (pb 16) du plasmide pSP 72.

Ce plasmide a été obtenu par clonage des différents fragments dans les régions indiquées du plasmide pSP 72. Il est entendu que des fragments équivalents peuvent être obtenus par l'homme du métier à partir d'autres sources.

Le pE2Gal plasmide est ensuite co-transfecté avec l'ADN du virus H2dl802 dépourvu de la région E2 (Rice et al. J. Virol. 56 (1985) 767) dans les cellules 293, en présence de phosphate de calcium. Le virus recombinant est ensuite préparé comme décrit dans l'exemple 1. Ce virus porte, comme seul gène viral, le gène E2 de l'adénovirus Ad2 (figure 5). Son génome a une taille de 12 kb environ, ce qui permet l'insertion d'ADN hétérologue de très grande taille (jusqu'à 20 kb). Ainsi, l'homme du métier peut aisément remplacer le gène LacZ par tout autre gène thérapeutique tels que ceux mentionnés plus haut. Par ailleurs, ce virus comporte certaines séquences issues du plasmide intermédiaire, qui peuvent être éliminées par les techniques classiques de biologie moléculaires si nécessaire.

### Exemple 4

Cet exemple décrit la construction de lignées cellulaires complémentantes pour les régions E1, E2 et/ou E4 des adénovirus. Ces lignées permettent la construction d'adénovirus recombinants selon l'invention délétés pour ces régions, sans avoir recours à un virus helper. Ces virus sont obtenus par recombinaison in vivo, et peuvent comporter des séquences hétérologues importantes.

Dans les lignées cellulaires décrites, les régions E2 et E4, potentiellement cytotoxiques, sont placées sous le controle d'un promoteur inductible : le LTR de MMTV (Pharmacia), qui est induit par la dexaméthasone, soit natif, soit la forme minimale décrite dans PNAS 90 (1993) 5603; ou le système repressible par la tétracycline décrit par Gossen et Büjard (PNAS 89 (1992) 5547). Il est entendu que d'autres promoteurs peuvent être utilisés, et notamment des variants du LTR de MMTV portant par exemple des régions hétérologues de régulation (région "enhancer" notamment). Les lignées de l'invention ont été construites par transfection des cellules correspondantes, en présence de phosphate de calcium, par un fragment d'ADN portant les gènes indiqués (régions d'adénovirus et/ou gène du récepteur aux glucocorticoïdes) sous le controle d'un promoteur de la transcription et d'un terminateur (site de polyadénylation). Le terminateur peut être soit le terminateur naturel du gène transfecté, soit un terminateur différent comme par exemple le terminateur du messager précoce du virus SV40. Avantageusement, le fragment d'ADN porte également un gène permettant la sélection des cellules transformées, et par exemple le gène de la résistance à la généticine. Le gène de résistance peut également être porté par un fragment d'ADN différent, co-transfecté avec le premier.

Après transfection, les cellules tranformées sont sélectionnées et leur ADN estvanalysé pour vérifier l'intégration du fragment d'ADN dans le génome.

Cette technique permet d'obtenir les lignées cellulaires suivantes :
1. Cellules 293 possédant le gène de la 72K de la région E2 d'Ad5 sous controle du LTR de MMTV;
2. Cellules 293 possèdant le gène de la 72K de la région E2 d'Ad5 sous controle du LTR de MMTV et le gène du récepteur aux glucocorticoïdes;
3. Cellules 293 possédant le gène de la 72K de la région E2 d'Ad5 sous controle du LTR de MMTV et la région E4 sous controle du LTR de MMTV;
4. Cellules 293 possèdant le gène de la 72K de la région E2 d'Ad5 sous controle du LTR de MMTV, la région E4 sous controle du LTR de MMTV, et le gène du récepteur aux glucocorticoïdes;
5. Cellules 293 possèdant la région E4 sous controle du LTR de MMTV;
6. Cellules 293 possèdant la région E4 sous controle du LTR de MMTV et le gène du récepteur aux glucocorticoïdes;
7. Cellules gm DBP6 possédant les régions E1A et E1B sous controle de leur propre promoteur;
8. Cellules gm DBP6 possédant les régions E1A et E1B sous controle de leur propre promoteur et la région E4 sous controle du LTR de MMTV.

### Exemple 5

Cet exemple décrit la préparation d'adénovirus recombinants défectifs selon l'invention dont le génome est délété des gènes E1, E3 et E4. Selon un mode avantageux de réalisation, illustré dans cet exemple et dans l'exemple 3 notamment, le génome des adénovirus recombinants de l'invention est modifié de sorte que les gènes E1 et E4 au moins soient non-fonctionnels. De tels adénovirus possèdent tout d'abord une capacité d'incorporation de gènes hétérologues importante. Par ailleurs, ces vecteurs présentent une sécurité élevée en raison de la délétion de la région E4, qui est impliquée dans la régulation de l'expression des gènes tardifs, dans la stabilité des ARN nucléaires tardifs, dans l'extinction de l'expression des protéines de la cellule hôte et dans l'efficacité de la réplication de l'ADN viral. Ces vecteurs possèdent donc un bruit de fond de transcription et une expression de gènes viraux très réduits. Enfin, de manière particulièrement avantageuse, ces vecteurs peuvent être produits à des titres comparables aux adénovirus sauvages.

Ces adénovirus ont été préparés à partir du plasmide pPY55, portant la partie droite modifiée du génome de l'adénovirus Ad5, soit par co-transfection avec un plasmide helper (voir également exemples 1, 2 et 3), soit au moyen d'une lignée complémentante (exemple 4).

### 5.1 Construction du plasmide pPY55

### a) Construction du plasmide pPY32

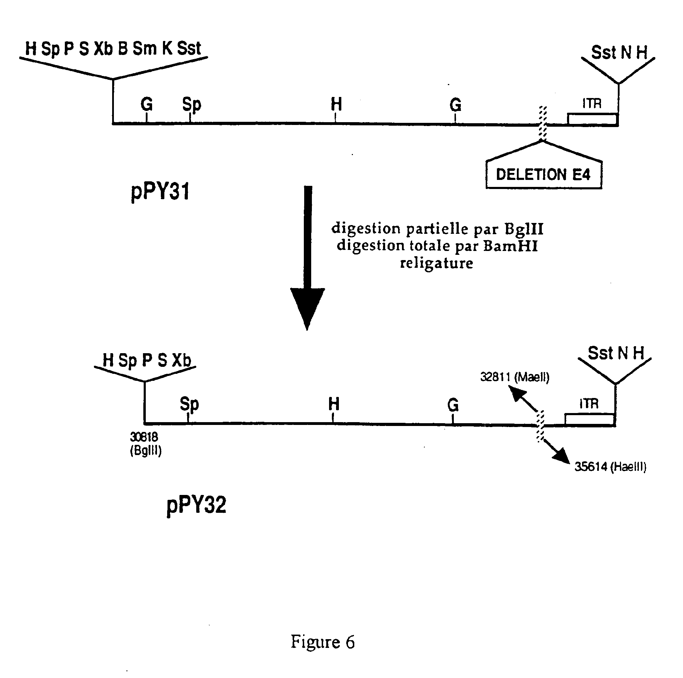

Le fragment AvrII-BcII du plasmide pFG144 [F.L. Graham et al. EMBO J. 8 (1989) 2077-2085], correspondant à l'extrémité droite du génome de l'adénovirus Ad5, a d'abord été cloné entre les sites XbaI et BamHI du vecteur pIC19H, préparé à partir d'un contexte dam-. Ceci génère le plasmide pPY23. Une caractéristique intéressante du plasmide pPY23 est que le site SalI provenant du multisite de clonage du vecteur pIC19H reste unique et qu'il est localisé à coté de l'extrémité droite du génome de l'adénovirus Ad5. Le fragment HaeIII-SalI du plasmide pPY23 qui contient l'extrémité droite du génome de l'adénovirus Ad5, à partir du site HaeIII localisé en position 35614, a ensuite été cloné entre les sites EcoRV et XhoI du vecteur pIC20H, ce qui génère le plasmide pPY29. Une caractéristique intéressante de ce plasmide est que les sites XbaI et ClaI provenant du multisite de clonage du vecteur pIC20H sont localisés à coté de la jonction EcoRV/HaeIII résultant du clonage. De plus cette jonction modifie le contexte nucléotidique immédiatement adjacent au site ClaI qui est maintenant devenu méthylable dans un contexte dam+. Le fragment XbaI(30470)-MaeII(32811) du génome de l'adénovirus Ad5 a ensuite été cloné entre les sites XbaI et ClaI du plasmide pPY29 préparé à partir d'un contexte dam-, ce qui génère le plasmide pPY30. Le fragment SstI du plasmide pPY30, qui correspond à la séquence du génome de l'adénovirus Ad5 du site SstI en position 30556 jusqu'à l'extrémité droite a enfin été cloné entre les sites SstI du vecteur pIC20H, ce qui génère le plasmide pPY31, dont une carte de restriction de l'insert localisé entre les sites HindIII est donnée à la Figure 6.

Le plasmide pPY32 a été obtenu après digestion partielle du plasmide pPY31 par BglII, suivie d'une digestion totale par BamHI, puis religature. Le plasmide pPY32 correspond donc à la délétion du génome de l'adénovirus Ad5 située entre le site BamHI du plasmide pPY31 et le site BglII localisé en position 30818. Une carte de restriction du fragment HindIII du plasmide pPY32 est donnée à la Figure 6. Une caractéristique du plasmide pPY32 est qu'il possède des sites SalI et XbaI uniques.

### b) Construction du plasmide pPY47

Le fragment BamHI(21562)-XbaI(28592) du génome de l'adénovirus Ad5 a tout d'abord été cloné entre les sites BamHI et XbaI du vecteur plC19H préparé à partir d'un contexte dam-, ce qui génère le plasmide pPY17. Ce plasmide contient donc un fragment HindIII (26328)-BgIII(28133) du génome de l'adénovirus Ad5, qui peut être cloné entre les sites HindIII et BgIII du vecteur pIC20R, pour générer le plasmide pPY34. Une caractéristique de ce plasmide est que le site BamHI provenant du multisite de clonage est localisé à proximité immédiate du site HindIII(26328) du génome de l'adénovirus Ad5.

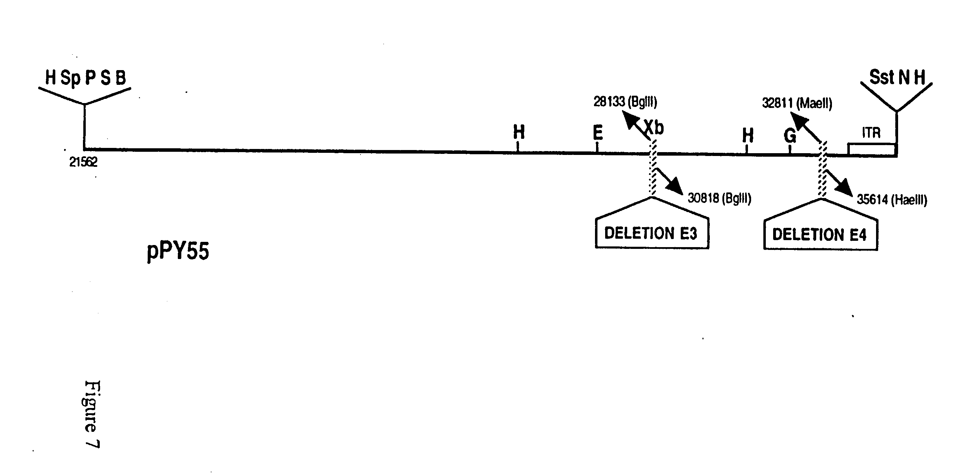

Le fragment BamHI (21562)-HindIII(26328) du génome de l'adénovirus Ad5 provenant du plasmide pPY17 a ensuite été cloné entre les sites BamHI et HindIII du plasmide pPY34, ce qui génère le plasmide pPY39. Le fragment BamHI-XbaI du plasmide pPY39 préparé à partir d'un contexte dam-, contenant la partie du génome de l'adénovirus Ad5 comprise entre les sites BamHI(21562) et BgIII(28133), a ensuite été cloné entre les sites BamHI et XbaI du vecteur pIC19H préparé à partir d'un contexte dam-. Ceci génère le plasmide pPY47 dont une caractéristique intéressante est que le site SalI provenant du multisite de clonage est localisé à proximité du site HindIII (figure 7).

### c) Construction du plasmide pPY55

Le fragment SalI-XbaI du plasmide pPY47 préparé à partir d'un contexte dam-, et qui contient la partie du génome de l'adénovirus Ad5 allant du site BamHI(21562) jusqu'au site BglII(28133), a été cloné entre les sites SalI et XbaI du plasmide pPY32, ce qui génère le plasmide pPY55. Ce plasmide est directement utilisable pour l'obtention d'adénovirus recombinants au moins délétés pour la région E3 (délétion entre les sites BgIII localisés aux positions 28133 et 30818 du génome de l'adénovirus Ad5) et pour la région E4 dans son intégralité (délétion entre les sites MaeII(32811) et HaeIII(35614) du génome de l'adénovirus Ad5 (figure 7).

### 5.2 Préparation des adenovirus comprenant au moins une délétion dans la région E4, et, de préférence, au moins dans les régions E1 et E4.

### a) Préparation par co-transfection avec un virus helper E4 dans les cellules 293

Le principe repose sur la transcomplémentation entre un "mini-virus" (virus helper) exprimant la région E4 et un virus recombinant délété au moins pour E3 et E4. Ces virus sont obtenus soit par ligature in vitro, soit après recombinaison in vivo, selon les stratégies suivantes :
(i) L'ADN du virus Ad-dl324 (Thimmappaya et al., Cell 31 (1982) 543) et le plasmide pPY55, tous deux digérés par BamHI, sont d'abord ligaturés in vitro, puis cotransfectés avec le plasmide pEAGaI (décrit dans l'exemple 2) dans les cellules 293.
(ii) L'ADN du virus Ad-dl324 digéré par EcoRI et le plasmide pPY55 digéré par BamHI sont contranfectés, avec le plasmide pE4Gal, dans les cellules 293.
(iii) L'ADN de l'adénovirus Ad5 et le plasmide pPY55, tous deux digérés par BamHI, sont ligaturés puis cotransfectés avec le plasmide pE4Gal dans les cellules 293.
(iv) L'ADN de l'adénovirus Ad5 digéré par EcoRI et le plasmide pPY55 digéré par BamHI sont cotransfectés avec le pEAGaI dans les cellules 293.

Les stratégies (i) et (ii) permettent de générer un adénovirus recombinant délété pour les régions E1, E3 et E4; les stratégies (iii) et (iv) permettent de générer un adénovirus recombinant délété pour les régions E3 et E4. Bien entendu, l'ADN d'un virus recombinant délété pour la région E1 mais exprimant un transgène quelconque peut être utilisé à la place de l'ADN du virus Ad-dl324 selon les stratégies (i) ou (ii), dans le but de générer un virus recombinant délété pour les régions E1, E3 et E4 et exprimant ledit transgène.

### b) Préparation au moyen de lignées cellulaires transcomplémentant les fonctions E1 et E4

Le principe repose ici sur le fait qu'une lignée cellulaire dérivée d'une lignée exprimant la région E1, par exemple la lignée 293, et exprimant également au moins les phases ouvertes ORF6 et ORF6/7 de la région E4 de l'adénovirus Ad5 sous contrôle d'un promoteur, par exemple inductible, est capable de transcomplémenter à la fois pour les régions E1 et E4 de l'adénovirus Ad5. De telles lignées ont été décrites dans l'exemple 4.

Un virus recombinant délété pour les régions E1, E3 et E4 peut donc être obtenu par ligation in vitro ou par recombinaison in vivo selon les protocoles décrits ci-dessus. Quelque soit le protocole utilisé pour générer les virus délétés au moins pour la région E4, un effet cytopathique (indiquant la production de virus recombinants) a été observé après transfection dans les cellules utilisées. Les cellules ont ensuite été récoltées, disruptées par trois cycles de congélation-décongélation dans leur surnageant, puis centrifugées à 4000 rpm pendant 10 minutes. Le surnageant ainsi obtenu a ensuite été amplifié sur culture cellulaire fraiche (cellules 293 pour les protocoles a) et cellules 293 exprimant la région E4 pour le protocole b)). Les virus ont alors été purifiés à partir de plages et leur ADN est analysé selon la méthode de Hirt (précité). Des stocks de virus sont ensuite préparés sur gradient de chlorure de césium.

### Exemple 6

Cet exemple décrit la préparation d'adénovirus recombinants défectifs selon l'invention dont le génome est délété des gènes E1, E3, L5 et E4. Ces vecteurs sont particulièrement avantageux puisque la région L5 code pour la fibre, qui est une protéine extrêment toxique pour la cellule.

Ces adénovirus ont été préparés à partir du plasmide p2, portant la partie droite modifiée du génome de l'adénovirus Ad5, par co-transfection avec différents plasmides helper. Ils peuvent également être préparés au moyen d'une lignée complémentante.

### 6.1 Construction du plasmide p2

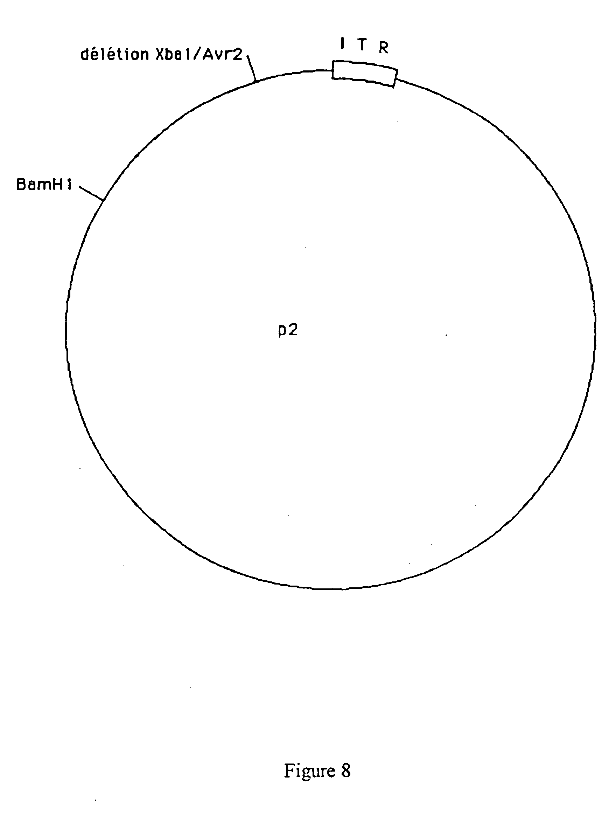

Ce plasmide contient toute la région droite du génome de l'adénovirus Ad5, à partir du site BamHI (21562), de laquelle a été délété le fragment compris entre les sites XbaI (28592) et AvrII (35463), portant les gènes E3, L5 et E4. Le plasmide p2 a été obtenu par clonage et ligature des fragments suivants dans le plasmide pIC19R linéarisé par BamHI et déphosphorylé (voir figure 8) :
- fragment du génome de l'adénovirus Ad5 compris entre les sites BamHI (21562) et XbaI (28592), et
- extrémité droite du génome de l'adénovirus Ad5 (contenant l'ITR droite), à partir du site AvrII (35463), jusqu'au site BclI (compatible BamHI).

### 6.2. Construction d'un plasmide helper (pITRL5-E4) portant le gène L5

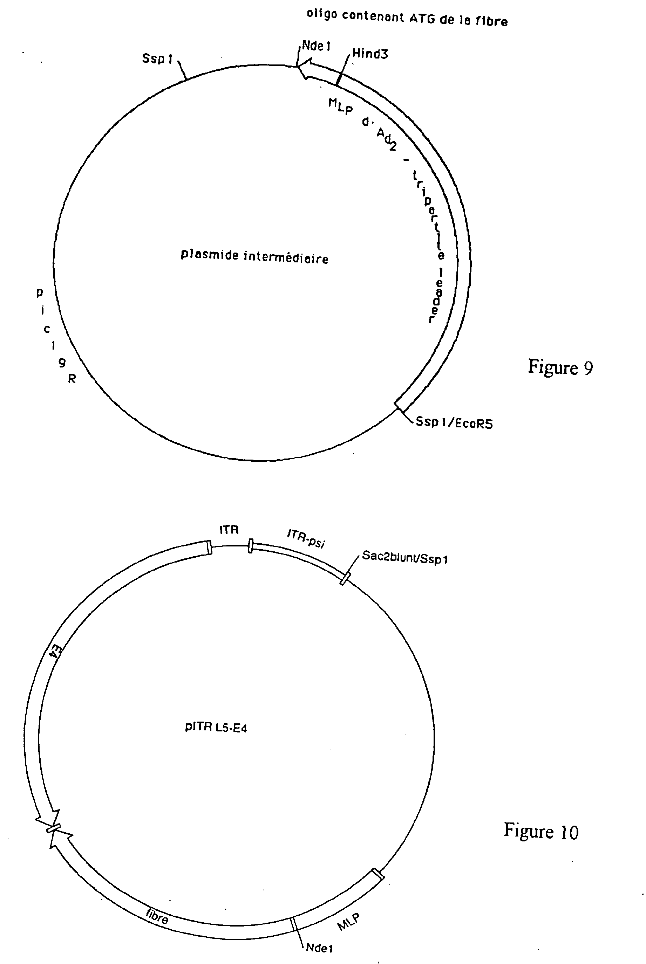

Le plasmide helper pITRL5-E4 apporte en trans les gènes E4 et L5. Il correspond au plasmide pE4Gal décrit dans l'exemple 2, contenant en plus la région L5 codant pour la fibre sous controle du promoteur MLP de l'adénovirus Ad2. Le plasmide pITRL5-E4 a été construit de la manière suivante (figures 9 et 10) :
Un oligonucléotide de 58 pb contenant, dans le sens 5'-3', un site HindIII, l'ATG de la fibre et la séquence codante de la fibre jusqu'au site NdeI en position 31089 du génome de l'adénovirus Ad5 a été synthétisé. La séquence de cet oligonucléotide est donnée ci-dessous, dans l'orientation 5'-3' :
   AAGCTTATGAAGCGCGCAAGACCGTCTGAAGATACCTTAACCCCGTGTATCCATATG
   Les sites HindIII, en 5', et NdeI, en 3', sont soulignés en simple, l'ATG de la fibre est souligné en double.
Un fragment SspI-HindIII contenant la séquence du promoteur MLP suivie du tripartite leader de l'adénovirus Ad2 a été isolé à partir du plasmide pMLP10 (Ballay et al., (1987) UCLA Symposia on molecular and cellular biology, New séries, Vol 70, Robinson et al (Eds) New-York, 481). Ce fragment a été inséré avec l'oligonucléotide de 58 pb décrit ci-dessus entre les sites NdeI et EcoRV du plasmide pIC19P, pour donner un plasmide intermédiaire (voir figure 9). Le fragment SacII (rendu blunt)-NdeI du plasmide pE4Gal (exemple 2) a ensuite été introduit dans le plasmide intermédiaire entre les sites SspI et NddeI pour générer le plasmide pITRL5-E4 (figure 10).

### 6.3 Préparation des adenovirus recombinants défectifs comprenant une délétion dans les régions E1, E3, L5 et E4.

### a) Préparation par co-transfection avec un virus helper dans les cellules 293

Le principe repose sur la transcomplémentation entre un "mini-virus" (virus helper) exprimant la région L5 ou les régions E4 et L5 et un virus recombinant délété au moins pour E3, E4 et L5.

Ces virus ont été obtenus soit par ligature in vitro, soit après recombinaison in vivo, selon les stratégies suivantes :
(i) L'ADN du virus Ad-dl324 (Thimmappaya et al., Cell 31 (1982) 543) et le plasmide p2, tous deux digérés par BamHI, ont tout d'abord été ligaturés in vitro, puis cotransfectés avec le plasmide helper pITRL5-E4 (exemple 6.2.) dans les cellules 293.
(ii) L'ADN du virus Ad-d1324 digéré par EcoRI et le plasmide p2 digéré par BamHI sont contranfectés, avec le plasmide pITRL5-E4, dans les cellules 293.
(iii) L'ADN de l'adénovirus Ad5 et le plasmide p2, tous deux digérés par BamHI, sont ligaturés puis cotransfectés avec le plasmide pITRL5-E4 dans les cellules 293.
(iv) L'ADN de l'adénovirus Ad5 digéré par EcoRI et le plasmide p2 digéré par BamHI sont cotransfectés avec le pITRL5-E4 dans les cellules 293.

Les stratégies (i) et (ii) permettent de générer un adénovirus recombinant délété pour les régions E1, E3, L5 et E4; les stratégies (iii) et (iv) permettent de générer un adénovirus recombinant délété pour les régions E3, L5 et E4. Bien entendu, l'ADN d'un virus recombinant délété pour la région E1 mais exprimant un transgène quelconque peut être utilisé à la place de l'ADN du virus Ad-dl324 selon les stratégies (i) ou (ii), dans le but de générer un virus recombinant délété pour les régions E1, E3, L5 et E4 et exprimant ledit transgène.

Les protocoles décrits ci-dessus peuvent également être mis en oeuvre avec un virus helper ne portant que la région L5, en utilisant une lignée cellulaire capable d'exprimer les régions E1 et E4 de l'adénovirus, telle que décrite dans l'exemple 4.

Par ailleurs, il est également possible d'utiliser une lignée complémentante capable d'exprimer les régions E1, E4 et L5, de façon à s'affranchir totalement de l'emploi d'un virus helper.

Après la transfection, les virus produits sont récupérés, amplifiés et purifiés dans les conditions décrites dans l'exemple 5.

## Revendications

1. Adénovirus recombinant défectif **caractérisé en ce qu'**il comprend :
- les séquences ITR,
- une séquence permettant l'encapsidation,
- une séquence d'ADN hétérologue
- une région portant le gène E2, et
- dans lequel le gène E1 et au moins le gène E4 sont non fonctionnels.

2. Adénovirus selon la revendication 1 **caractérisé en ce qu'**il comprend un gène E3 fonctionnel sous controle d'un promoteur hétérologue.

3. Adénovirus selon l'une des revendications précédentes **caractérisé en ce que** la séquence d'ADN hétérologue comporte un ou plusieurs gènes thérapeutiques et/ou un ou plusieurs gènes codant pour des peptides antigéniques.

4. Adénovirus selon la revendication 3 **caractérisé en ce que** le gène thérapeutique est choisi parmi les gènes codant pour des enzymes, des dérivés sanguins, des hormones, des lymphokines (interleukines, interférons, TNF, etc), des facteurs de croissance, des neurotransmetteurs ou leurs précurseurs ou enzymes de synthèse, des facteurs trophiques (BDNF, CNTF, NGF, IGF, GMF, aFGF, bFGF, NT3, NT5, etc), des apolipoprotéines (ApoAI, ApoAIV, ApoE, etc), la dystrophine ou une minidystrophine, des gènes suppresseurs de tumeurs ou des gènes codant pour des facteurs impliqués dans la coagulation (Facteurs VH, VM, IX, etc).

5. Adénovirus selon la revendication 3 **caractérisé en ce que** le gène thérapeutique est un gène ou une séquence antisens, dont l'expression dans la cellule cible permet de contrôler l'expression de gènes ou la transcription d'ARNm cellulaires.

6. Adénovirus selon la revendication 3 **caractérisé en ce que** le gène code pour un peptide antigénique capable de générer chez l'homme une réponse immunitaire contre des microorganismes ou des virus.

7. Adénovirus selon la revendication 6 **caractérisé en ce que** le gène code pour un peptide antigénique spécifique du virus d'Epstein Barr, du virus MV, du virus de l'hépatite B ou du virus de la pseudo-rage.

8. Adénovirus selon la revendication 3, **caractérisé en ce que** le gène code un peptide antigénique spécifique de tumeurs.

9. Adénovirus selon l'une des revendications précédentes **caractérisé en ce que** la séquence d'ADN hétérologue comprend également des séquences permettant l'expression du gène thérapeutique et/ou du gène codant pour le peptide antigénique dans la cellule infectée.

10. Adénovirus selon l'une des revendications précédentes **caractérisé en ce que** la séquence d'ADN hétérologue comprend, en amont du gène thérapeutique, une séquence signal dirigeant le produit thérapeutique synthétisé dans les voies de sécrétion de la cellule cible.

11. Lignée cellulaire qui peut être infectée par un adénovirus et qui comprend, intégrées dans son génome, les fonctions nécessaires à la complémentation d'un adénovirus recombinant défectif selon les revendications 1 à 10, **caractérisée en ce qu'**elle comporte dans son génome au moins les gènes E1 et E4 d'un adénovirus et que le gène E4 est placé sous le contrôle d'un promoteur inductible.

12. Lignée cellulaire selon la revendication 11 **caractérisée en ce qu'**elle comporte en outre le gène du récepteur aux glucocorticoïdes.

13. Lignée cellulaire selon la revendication 11 **caractérisée en ce que** le promoteur inductible est le promoteur LTR de MMTV.

14. Lignée cellulaire selon les revendications 11 à 13 **caractérisée en ce qu'**elle est obtenue à partir de la lignée 293.

15. Composition pharmaceutique comprenant au moins un adénovirus recombinant défectif selon l'une des revendications 1 à 10.

16. Composition pharmaceutique selon la revendication 15 comprenant un véhicule pharmaceutiquement acceptable pour une formulation injectable.

## Claims

1. Defective recombinant adenovirus **characterized in that** it comprises:
- the ITR sequences,
- a sequence permitting the encapsulation,
- a heterologous DNA sequence,
- a region carrying the E2 gene, and
- in which the E1 gene and at least the E4 gene are non-functional.

2. Adenovirus according to Claim 1, **characterized in that** it comprises a functional gene E3 under the control of a heterologous promoter.

3. Adenovirus according to either of the preceding claims, **characterized in that** the heterologous DNA sequence contains one or more therapeutic genes and/or one or more genes encoding antigenic peptides.

4. Adenovirus according to Claim 3, **characterized in that** the therapeutic gene is chosen from the genes encoding enzymes, blood derivatives, hormones, lymphokines (interleukins, interferons, TNF and the like), growth factors, neurotransmitters or their precursors or synthetic enzymes, trophic factors (BDNF, CNTF, NGF, IGF, GMF, aFGF, bFGF, NT3, NT5 and the like), apolipoproteins (ApoAI, ApoAIV, ApoE and the like), dystrophin or a minidystrophin, tumour suppressor genes or genes encoding factors involved in coagulation (Factors VH, VM, IX and the like).

5. Adenovirus according to Claim 3, **characterized in that** the therapeutic gene is an antisens gene or sequence whose expression in the target cell makes it possible to control the expression of genes or the transcription of cellular mRNAs.

6. Adenovirus according to Claim 3, **characterized in that** the gene encodes an antigenic peptide capable of generating an immune response in man against microorganisms or viruses.

7. Adenovirus according to Claim 6, **characterized in that** the gene encodes an antigenic peptide specific for the Epstein Barr virus, the MV virus, the hepatitis B virus or the pseudo-rabies virus.

8. Adenovirus according to Claim 3, **characterized in that** the gene encodes an antigenic peptide specific for tumours.

9. Adenovirus according to one of the preceding claims, **characterized in that** the heterologous DNA sequence also comprises sequences permitting the expression of the therapeutic gene and/or of the gene encoding the antigenic peptide in the infected cell.

10. Adenovirus according to one of the preceding claims, **characterized in that** the heterologous DNA sequence comprises, upstream of the therapeutic gene, a signal sequence directing the therapeutic product synthesized in the secretory pathways of the target cell.

11. Cell line which can be infected by an adenovirus and which comprises, integrated into its genome, the functions necessary for the complementation of a defective recombinant adenovirus according to Claims 1 to 10, **characterized in that** it contains, in its genome, at least the E1 and E4 genes from an adenovirus and that the E4 gene is placed under the control of an inducible promoter.

12. Cell line according to Claim 11, **characterized in that** it contains, in addition, the gene for the glucocorticoid receptor.

13. Cell line according to Claim 11, **characterized in that** the inducible promoter is the LTR promoter of MMTV.

14. Cell line according to Claims 11 to 13, **characterized in that** it is obtained from the line 293.

15. Pharmaceutical composition comprising at least one defective recombinant adenovirus according to one of Claims 1 to 10.

16. Pharmaceutical composition according to Claim 15, comprising a vehicle pharmaceutically acceptable for an injectable formulation.

## Patentansprüche

1. Defektives rekombinantes Adenovirus, **dadurch gekennzeichnet, dass** es umfasst:
- ITR-Sequenzen,
- eine Sequenz, welche die Verkapselung ermöglicht,
- eine heterologe DNA-Sequenz,
- eine Region, welche das Gen E2 trägt und
- in dem das Gen E1 und mindestens das Gen E4 nicht funktionell sind.

2. Adenovirus nach Anspruch 1, **dadurch gekennzeichnet, dass** es ein funktionelles E3-Gen unter Kontrolle eines heterologen Promotors umfasst.

3. Adenovirus nach einem der vorherigen Ansprüche, **dadurch gekennzeichnet, dass** die heterologe DNA-Sequenz ein oder mehrere therapeutische Gene und/oder ein oder mehrere Gene aufweist, die für antigene Peptide kodieren.

4. Adenovirus nach Anspruch 3, **dadurch gekennzeichnet, dass** das therapeutische Gen ausgewählt ist aus Genen, die für Enzyme, Blutderivate, Hormone, Lymphokine (Interleukine, Interferone, TNF, etc.), Wachstumsfaktoren, Neurotransmitter oder deren Vorläufer oder Syntheseenzyme, trophische Faktoren (BDNF, CNTF, NGF, IGF, GMF, aFGF, bFGF, NT3, NT5, etc.), Apolipoproteine (ApoAI, ApoAIV, ApoE, etc.), Dystrophin oder ein Minidystrophin kodieren, Tumorsuppressorgenen oder Genen, die für an der Koagulation beteiligte Faktoren (Faktoren VH, VM, IX, etc.) kodieren.

5. Adenovirus nach Anspruch 3, **dadurch gekennzeichnet, dass** das therapeutische Gen ein Gen oder eine Antisense-Sequenz ist, deren Expression in der Zielzelle es ermöglicht, die Expression von Genen oder die Transkription von mRNA der Zelle zu kontrollieren.

6. Adenovirus nach Anspruch 3, **dadurch gekennzeichnet, dass** das Gen für ein antigenes Peptid kodiert, das beim Menschen eine Immunantwort gegen Mikroorganismen oder Viren erzeugen kann.

7. Adenovirus nach Anspruch 6, **dadurch gekennzeichnet, dass** das Gen für ein spezifisches antigenes Peptid des Epstein-Barr-Virus, des MV-Virus, des Hepatitis B-Virus oder des Pseudorabiesvirus kodiert.

8. Adenovirus nach Anspruch 3, **dadurch gekennzeichnet, dass** das Gen für ein tumorspezifisches antigenes Peptid kodiert.

9. Adenovirus nach einem der vorherigen Ansprüche, **dadurch gekennzeichnet, dass** die heterologe DNA-Sequenz ebenfalls Sequenzen umfasst, welche die Expression des therapeutischen Gens und/oder des Gens, das für das antigene Peptid kodiert in der infizierten Zelle ermöglichen.

10. Adenovirus nach einem der vorherigen Ansprüche, **dadurch gekennzeichnet, dass** die heterologe DNA-Sequenz stromaufwärts des therapeutischen Gens eine Signalsequenz umfasst, die das synthetisierte therapeutische Produkt in die sektretorischen Bahnen der Zielzelle leitet.

11. Zelllinie, die durch ein Adenovirus infiziert werden kann und die, in ihrem Genom integriert, die Funktionen umfasst, welche zur Komplementierung eines rekombinanten defektiven Adenovirus nach den Ansprüchen 1 bis 10 erforderlich sind, **dadurch gekennzeichnet, dass** sie in ihrem Genom mindestens die Gene E1 und E4 eines Adenovirus aufweist und dass das Gen E4 unter die Kontrolle eines induzierbaren Promotors gestellt ist.

12. Zelllinie nach Anspruch 11, **dadurch gekennzeichnet, dass** sie ferner das Gen für den Glucocorticoidrezeptor aufweist.

13. Zelllinie nach Anspruch 11, **dadurch gekennzeichnet, dass** der induzierbare Promotor der LTR Promotor von MMTV ist.

14. Zelllinie nach den Ansprüchen 11 bis 13, **dadurch gekennzeichnet, dass** sie aus der Linie 293 erhalten wird.

15. Arzneimittelzusammensetzung, die mindestens ein defektives rekombinantes Adenovirus nach einem der Ansprüche 1 bis 10 aufweist.

16. Arzneimittelzusammensetzung nach Anspruch 15, die einen pharmazeutisch verträglichen Trägerstoff für eine injizierbare Formulierung aufweist.
